# FASCICULE DE BREVET EUROPEEN

(11) **EP 4 037 477 B1**
(45) Date de publication et mention de la délivrance du brevet: **24.06.2026**
(21) Numéro de dépôt: 19801955.6
(22) Date de dépôt: 03.10.2019
(51) Int. Cl.: A01K 1/01, A01K 23/00, A61B 10/00

(54) **BOITIER DE CAPTURE ET D'ANALYSE D'URINE D'UN ANIMAL, DISPOSITIF ET PROCÉDÉ LE METTANT EN OEUVRE**
GEHÄUSE ZUM AUFFANGEN UND ANALYSIEREN DES URINS EINES TIERES, VERWANDTES GERÄT UND VERFAHREN
ANIMAL URINE CAPTURE AND ANALYSIS BOX, CORRESPONDING APPARATUS AND METHOD OF USE

(43) Date de publication de la demande: 10.08.2022
(73) Titulaire: Invoxia, 92130 Issy les Moulineaux (FR)
(72) Inventeur: DAURENJOU, Philippe, 34980 MURLES (FR)
(74) Mandataire: Cornuejols, Georges
(86) Numéro de dépôt international: PCT/FR2019/052352
(87) Numéro de publication internationale: WO 2020/070454

(56) Documents cités:
- EP-A1- 0 749 685
- US-A- 4 084 937
- US-A- 5 353 743
- US-A- 5 518 003
- US-A1- 2013 053 729
- US-A1- 2014 213 934

## Description

### DOMAINE TECHNIQUE DE L'INVENTION

La présente invention concerne un boîtier de capture et d'analyse d'urine d'un animal, un dispositif et un procédé mettant en œuvre ce boîtier.

Elle s'applique notamment au domaine vétérinaire et, plus particulièrement, à la surveillance de la santé des chats et des chiens.

### ÉTAT DE LA TECHNIQUE

Les vétérinaires utilisent des bandelettes urinaires pour analyser les urines des animaux. Cependant, les rendez-vous s'échelonnant sur la journée de travail, l'animal n'est généralement pas à jeun, a déjà fait des efforts physiques et peut avoir bu. La qualité de l'urine, en termes physico-chimiques, est donc variable et source d'erreurs de diagnostic. Le stress de l'animal amené chez le vétérinaire vient également modifier les concentrations réelles de certains analytes.

On connaît des dispositifs de collecte d'urine animale pour propriétaires, constitués de cannes que l'utilisateur doit positionner sous l'animal pendant que celui-ci urine. Ces dispositifs imposent de surveiller l'animal pour être présent lorsqu'il urine. De plus, ces cannes perturbent les animaux et peuvent les inciter à retenir leur urine.

On connaît la demande de brevet américain US 2013/053729, la demande de brevet américain US 5 518 003 et la demande de brevet américain US 5 353 743 qui divulguent des dispositifs d'analyse d'urine pour animaux.

### OBJET DE L'INVENTION

La présente invention vise à remédier à tout ou partie de ces inconvénients.

À cet effet, selon un premier aspect, la présente invention vise un boîtier de capture et d'analyse d'urine d'un animal selon la revendication 1.

Grâce à ces dispositions, le boîtier collecte d'abord de l'urine puis, après basculement du boîtier, imprègne une bandelette avec l'urine collectée. Le durée d'imprégnation est ainsi maîtrisée, ce qui améliore sensiblement la qualité des analyses. Le boîtier peut être transporté auprès d'un vétérinaire et la bandelette peut être analysée par le propriétaire de l'animal, par exemple par comparaison avec des couleurs de témoin identifiant des quantités de composants dans l'urine résiduelle.

Dans des modes de réalisation :
- Le boîtier comporte une vanne qui obture ou ouvre le passage entre lesdits réceptacles ;
- la vanne est une vanne multivoie qui obture ou ouvre, de plus, une entrée d'urine dans le boîtier ;
- la vanne comporte un réservoir de retenue du premier jet d'urine pénétrant dans le boîtier ;
- le réservoir de retenue du premier jet d'urine est surmonté d'une cloison limitant l'écoulement de l'urine vers le deuxième réceptacle, dans la deuxième configuration du boîtier ;
- le premier réceptacle comporte au moins un compartiment latéral et une cloison de retenue d'urine dans un compartiment latéral lorsque le boîtier bascule dans la deuxième configuration ;
- la cloison de retenue d'urine dans le compartiment latéral présente une chicane ou un hauteur de débordement ;
- le boîtier comporte un couvercle transparent au moins en regard du support de bandelette, le couvercle étant mobile par rapport à une base comportant les réceptacles et/ou
- le couvercle porte des repères de repérage de la bandelette dans une image du couvercle.

Selon un deuxième aspect, l'invention vise un dispositif de collecte d'urine d'un animal prédéterminé, qui comporte :
- un boîtier de capture et d'analyse d'urine selon la revendication 1,
- un bac d'accueil de l'animal urinant et de retenue d'éléments inertes vis-à-vis de l'urine, sur un tamis configuré pour retenir les éléments inertes et laisser passer l'urine,
- une surface en pente, sous le tamis, pour rassembler l'urine en une zone de convergence menant à une entrée du boîtier de capture et d'analyse d'urine.

Grâce à ces dispositions, l'animal se sent comme sur une litière lorsqu'il urine dans le bac de retenue. De plus, l'urine prélevée provient d'au moins un jet postérieurs au premier jet souvent souillé au niveau du canal de l'urètre et support de récolte. L'urine est ainsi plus révélatrice des éventuels défauts de santé de l'animal. Enfin, le dispositif est facile à nettoyer puisqu'il suffit de faire passer de l'eau sur le même trajet que l'urine c'est-à-dire depuis les éléments inertes, sur le tamis, sur la pente et sur le moyen de capture et le moyen de prélèvement. Un propriétaire d'animal domestique peut ainsi procéder à un prélèvement d'urine sans l'aide d'un vétérinaire. Et ce prélèvement peut être de meilleure qualité que celle pratiquée par un vétérinaire car l'animal peut être à jeun, ne pas avoir fait d'efforts physiques, ne pas avoir bu et ne pas être stressé.

Dans des modes de réalisation, le moyen de capture de premier jet d'urine comporte un réservoir en dessous de la zone de convergence.

Grâce à ces dispositions, la gravité fait arriver le premier jet d'urine dans le réservoir, le prélèvement d'urine se faisant dans la zone de convergence.

Dans des modes de réalisation, le moyen de capture de premier jet d'urine comporte une vanne sur le passage de l'urine en aval de la zone de convergence, la vanne étant commandée pour changer de position et orienter l'urine vers une zone de prélèvement après qu'une certaine quantité d'urine ait traversé la vanne.

Grâce à ces dispositions, la zone de prélèvement peut être séparée de la zone de convergence.

Dans des modes de réalisation, le moyen de capture de premier jet d'urine comporte une matière absorbante configurée pour absorber l'urine.

Grâce à ces dispositions, l'urine du premier jet est absorbée et mécaniquement retenue dans la matière absorbante. Les risques d'écoulement de cette urine, lors de la manipulation du dispositif, sont donc réduits.

Dans des modes de réalisation, le moyen de capture de premier jet d'urine comporte une chicane.

Grâce à ces dispositions, l'urine du premier jet est retenue et empêchée de retourner dans la zone de convergence.

Dans des modes de réalisation, la surface en pente comporte, au moins en surface en contact avec l'urine, un matériau hydrophobe.

Grâce à ces dispositions, l'écoulement de l'urine est facilité et la vitesse de convergence de l'urine est plus stable, ce qui favorise l'arrivée en premier, dans la zone de convergence, de l'urine du premier jet.

Dans des modes de réalisation, la surface en pente hydrophobe comporte du PEHD grade 6.

Dans des modes de réalisation, la surface en pente comporte deux pentes convergeant vers une ligne d'intersection inclinée vers la zone de convergence.

Grâce à ces dispositions, la forme de la surface en pente favorise son nettoyage et la régularité de la vitesse d'écoulement de l'urine.

Dans des modes de réalisation, la pente de la surface en pente est comprise entre trois et cinq degrés d'angle.

Dans des modes de réalisation, la surface en pente présente une forme en accordéon dont les parties basses sont en pente vers une surface de collecte inclinée vers la zone de convergence.

Grâce à chacune de ces dispositions, la vitesse d'écoulement de l'urine est régulière.

Dans des modes de réalisation, les éléments inertes comportent des granules plastiques sphériques ou cylindrique hydrophobes.

Grâce à ces dispositions, l'urine est faiblement retenue par les éléments inertes et leur lavage est facilité.

Dans des modes de réalisation, le dispositif de collecte d'urine comporte un couvercle amovible pour enfermer les éléments inertes dans la bac de retenue.

Grâce à ces dispositions, le nettoyage des éléments inertes est facilité car ils ne risquent pas de s'échapper sous l'effet d'un jet d'eau.

Dans des modes de réalisation, le dispositif comporte deux bacs de hauteur identiques, l'un pour la retenue des éléments inertes, l'autre pour la retenue de litière.

Grâce à ces dispositions, lorsque l'on commute les positions respectives des bacs, l'un au-dessus de l'autre, l'animal conserve une même hauteur à franchir pour atteindre la litière ou les éléments inertes. Il n'est donc pas surpris et le risque qu'il n'urine pas est ainsi réduit.

Dans des modes de réalisation, chacun des deux bacs comporte des piétement internes de support de l'autre bac. Grâce à ces disposition la commutation des positions respectives des bacs, l'un au-dessus de l'autre, est facilitée.

Dans des modes de réalisation, le dispositif comporte un moyen de mesure de poids de l'animal prédéterminé et/ou de l'urine collectée avant prélèvement.

Grâce à ces dispositions, au moins une mesure indicative de l'état de santé de l'animal est obtenue par le dispositif. Le moyen de mesure de poids renseigne également sur le comportement de l'animal (fréquence de passage et durée sur le bac).

Dans des modes de réalisation, le dispositif comporte un moyen de mesure de température de l'animal et/ou de l'urine collectée.

Grâce à ces dispositions, au moins une mesure indicative de l'état de santé de l'animal est obtenue par le dispositif.

Dans des modes de réalisation, le dispositif comporte un moyen de décompte du nombre de passages pendant une durée prédéterminée et/ou de la durée de passage de l'animal dans le dispositif.

Grâce à ces dispositions, des difficultés intestinales ou urinaires de l'animal peuvent être révélées.

Dans des modes de réalisation, le boîtier, aussi appelé « récipient », est transparent et comporte un réservoir d'urine prélevée et un bouchon d'accès à ce réservoir par une bandelette portant des témoins comportant des réactifs.

Grâce à ces dispositions, une mesure de couleur et de clairance de l'urine peut être réalisée à travers le récipient et une bandelette peut être introduite directement dans le réservoir, par exemple par un vétérinaire souhaitant réaliser une analyse d'urine complémentaire.

Dans des modes de réalisation, le dispositif comporte une valve dans la zone de convergence et le récipient comporte un embout configuré pour provoquer l'ouverture de la valve et permettre l'écoulement par gravité de l'urine résiduelle vers le récipient.

Dans des modes de réalisation, le dispositif comporte une valve dans la zone de convergence et le récipient comporte un embout configuré pour provoquer l'ouverture de la valve et un moyen de mise en dépression du récipient pour pomper de l'urine résiduelle vers le récipient.

Grâce à chacune de ces dispositions, la zone de convergence retient l'urine résiduelle jusqu'à ce qu'une partie de l'urine résiduelle soit extraite vers le récipient.

Dans des modes de réalisation, le récipient est, au moins partiellement, transparent dans les longueurs d'onde visibles.

Grâce à ces dispositions, les caractéristiques physiques du contenu du récipient peuvent être observées, en particulier, la couleur de l'urine et la turbidité de l'urine.

Dans des modes de réalisation, le dispositif comporte, de plus, un moyen d'analyse de l'urine résiduelle prélevée par le moyen de prélèvement.

Grâce à ces dispositions, le propriétaire de l'animal peut procéder à une première analyse de l'urine et, en fonction des résultats de cette analyse, décider de consulter un vétérinaire.

Dans des modes de réalisation, le moyen d'analyse comporte un capteur de lumière pour capter la couleur d'au moins un témoin comportant au moins un réactif.

Grâce à ces dispositions, le moyen d'analyse peut évaluer au moins une quantité de composant dans l'urine résiduelle prélevée.

Dans des modes de réalisation, le capteur de lumière comporte un capteur d'image matriciel et une optique pour prendre au moins une image d'une bandelette comportant une pluralité de témoins.

Grâce à ces dispositions, les quantités de plusieurs composants dans l'urine prélevée peuvent être évaluées simultanément.

Dans des modes de réalisation, le moyen d'analyse comporte un moyen de compensation des variations d'éclairage de chaque témoin.

Grâce à ces dispositions, quelles que soient les variations de l'éclairage, naturel ou artificiel, des témoins, la couleur de chaque témoin est évaluée après compensation de ces variations.

Dans des modes de réalisation, le moyen d'analyse comporte un moyen d'évaluation de la couleur de l'urine prélevée.

Dans des modes de réalisation, le moyen d'analyse comporte un moyen d'évaluation de la clairance de l'urine prélevée.

Grâce à chacune de ces dispositions, des caractéristiques physiques de l'urine prélevée sont évaluées.

Dans des modes de réalisation, le moyen d'analyse comporte un moyen de comparaison :
- d'au moins une couleur évaluée avec au moins une valeur limite de couleur et/ou
- d'au moins une quantité évaluée de composant de l'urine avec au moins une valeur limite de quantité.

Grâce à ces dispositions, le propriétaire de l'animal peut être automatiquement prévenue d'une possible anomalie de la composition de l'urine et d'un potentiel problème de santé de l'animal. Il peut donc décider de consulter un vétérinaire sur la base d'éléments objectifs.

Dans des modes de réalisation, le moyen d'analyse comporte un moyen d'émission à distance de couleurs évaluées et/ou de résultats de comparaison des couleurs évaluées.

Dans des modes de réalisation, le moyen d'analyse comporte un moyen de réception depuis un émetteur distant, de valeurs limites de couleur et/ou de quantités de composant dans l'urine.

Grâce à chacune de ces dispositions, le praticien ou le vétérinaire, peut recevoir et/ou paramétrer l'analyse d'urine effectuée par le moyen d'analyse.

Selon un troisième aspect, l'invention vise un procédé d'utilisation d'un boîtier objet de l'invention ou un dispositif objet de l'invention, qui comporte :
- une étape de prise d'une image du boîtier,
- une étape d'identification d'une bandelette dans l'image captée,
- une étape de détermination de couleur de pastilles portées par la bandelette,
- une étape de comparaison d'au moins une couleur de pastille avec au moins une valeur limite de couleur, pour détecter une anomalie
- une étape d'identification d'au moins un compartiment retenant de l'urine, dans l'image captée,
- une étape de mesure de turbidité et/ou de couleur de l'urine retenue dans au moins un compartiment et
- une étape de comparaison de la turbidité mesurée et/ou de la couleur mesurée avec au moins une valeur limite pour détecter une anomalie et
- une étape d'affichage de chaque anomalie détectée.

Dans des modes de réalisation, le procédé comporte, de plus :
- une étape d'initialisation de valeurs limites de détection d'anomalies et
- une étape de réévaluation d'au moins une valeur limite en fonction des valeurs mesurées pour l'animal considéré.

Dans des modes de réalisation, le procédé comporte, de plus :
- une première étape de détermination de comportement de l'animal vis-à-vis du boîtier, par exemple heure, durée et fréquence de passage,
- une étape d'initialisation de valeurs limites relatives au comportement de l'animal,
- une deuxième étape de mesures de valeurs représentatives de comportement de l'animal et
- une étape de comparaison des mesures avec les valeurs limite, pour détecter une anomalie.

Dans des modes de réalisation, le procédé comporte, de plus :
- une première étape de détermination de poids de l'animal et/ou de poids d'au moins une miction de l'animal,
- une étape d'initialisation de valeurs limites relatives au poids déterminé,
- une deuxième étape de mesures de poids de l'animal et/ou de poids d'au moins une miction de l'animal et
- une étape de comparaison des mesures avec les valeurs limite, pour détecter une anomalie.

Les avantages, buts et caractéristiques particulières de ce procédé étant similaires à ceux du boîtier et/ou du dispositif objets de l'invention, ils ne sont pas rappelés ici.

### BRÈVE DESCRIPTION DES FIGURES

D'autres avantages, buts et caractéristiques de la présente invention ressortiront de la description qui va suivre, faite dans un but explicatif et nullement limitatif en regard du dessin annexé, dans lequel :
- la figure 1 est une vue en coupe d'un premier mode de réalisation particulier, non revendiqué, d'un dispositif de collecte d'urine,
- la figure 2 est une vue de dessus du dispositif illustré en figure 1,
- les figures 3 et 4 sont des vues en coupe du dispositif illustré en figures 1 et 2,
- la figure 5 est une vue en perspective du dispositif illustré en figures 1 à 4,
- la figure 6 représente un nuancier de comparaison de couleurs de témoins comportant des réactifs,
- la figure 7 représente, sous forme de logigramme, des étapes d'un procédé non revendiqué de prélèvement d'urine,
- la figure 8 représente, sous forme d'un schéma bloc, un moyen d'analyse d'un prélèvement d'urine,
- la figure 9 représente, sous forme d'un logigramme, des étapes d'analyse d'un prélèvement d'urine,
- la figure 10 est une vue en perspective d'un mode de réalisation particulier du dispositif objet de l'invention,
- la figure 11 est une vue de dessus d'une partie inférieure du dispositif illustré en figure 10,
- la figure 12 est une vue en élévation d'une assise du dispositif illustré en figures 10 et 11,
- la figure 13 est une vue de dessus de l'assise illustrée en figure 12,
- les figures 14 à 16 sont, respectivement, des vues en élévation, en coupe et de dessus, d'un boîtier amovible de capture et de prélèvement d'urine,
- les figures 17 et 18 sont, respectivement, des vues de dessus et de côté d'une base du boîtier illustré en figures 14 à 16,
- la figure 19 représente, de côté, une bride du boîtier illustré en figures 14 à 18 et
- la figure 20 est une vue de dessus d'un couvercle du boîtier illustré en figures 14 à 19.

### DESCRIPTION D'EXEMPLES DE RÉALISATION DE L'INVENTION

La présente description est donnée à titre non limitatif, chaque caractéristique d'un mode de réalisation pouvant être combinée à toute autre caractéristique de tout autre mode de réalisation de manière avantageuse. On note, dès à présent, que les figures ne sont pas toujours à l'échelle.

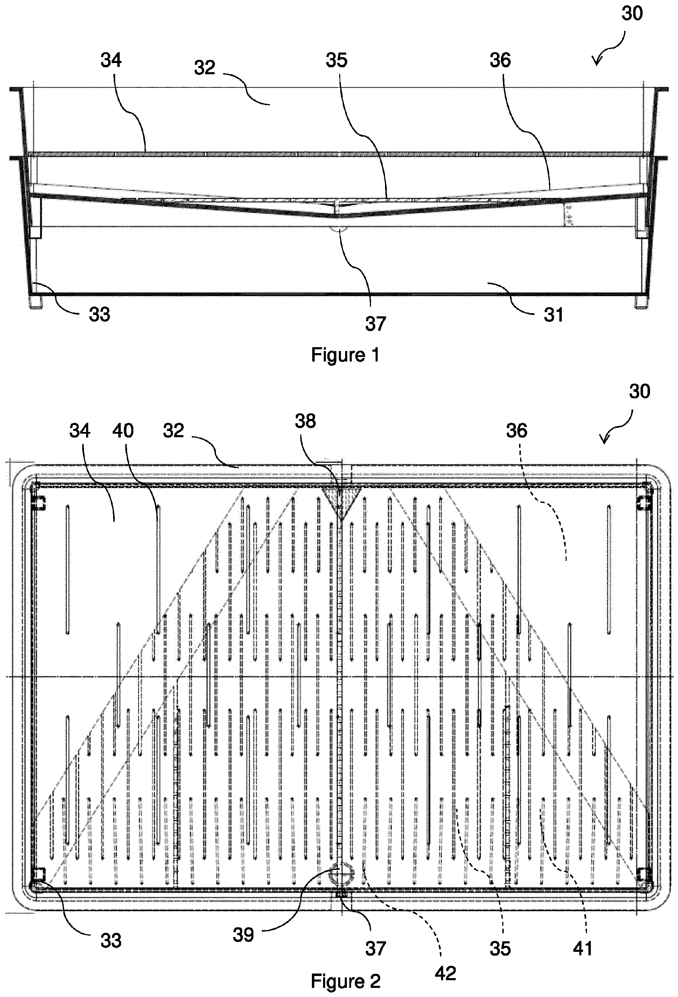

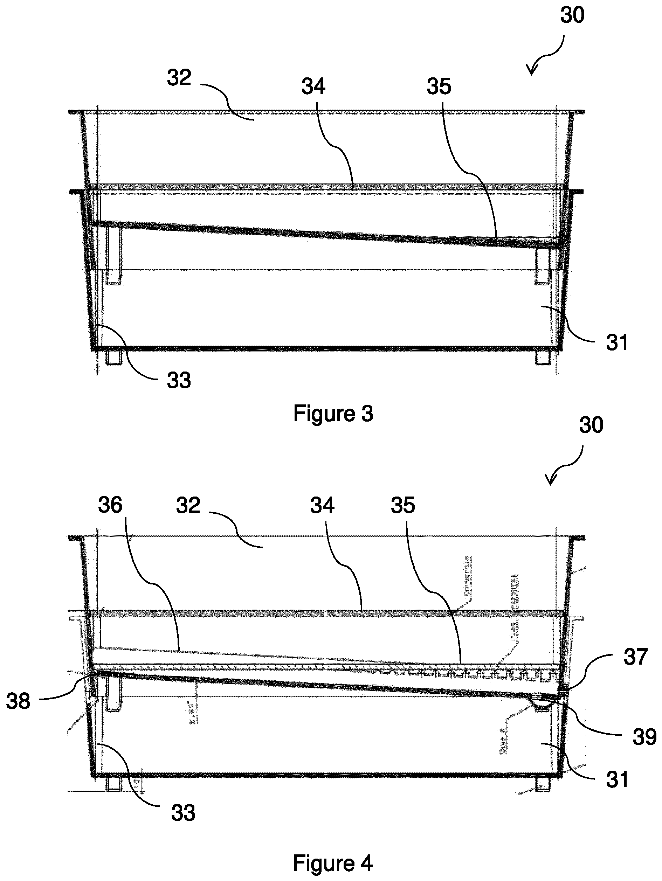

On observe, en figures 1 à 5, un dispositif 30 de collecte d'urine non revendiqué ici, qui comporte, dans sa configuration de collecte d'urine, un bac inférieur 31 et un bac supérieur 32. Le bac inférieur 31 est destiné à recevoir une litière afin qu'un animal prédéterminé, notamment un chat ou un chien, y urine. Dans la configuration la plus courante, hors collecte d'urine, on commute, par rapport à ce qui est représenté en figures 1 à 5, les positions respectives des bacs 31 et 32 afin que l'animal puisse monter dans le bac 31 qui se trouve alors au-dessus du bac 32.

Les bacs 31 et 32 comportent préférentiellement des piétements 33 formant support de l'autre bac, respectivement 32 et 31 de telle manière que, dans les deux configurations de superposition des bac, l'animal ait la même hauteur à atteindre pour uriner. On évite ainsi de surprendre l'animal et de le dissuader de monter dans la bac le plus haut pour y uriner.

Le bac 32 supporte un tamis 35 retenant des éléments (non représentés) inertes vis-à-vis de l'urine et laissant passer l'urine à travers des fentes 41. Lors du lavage des éléments inertes et du dispositif, le bac 32 est muni d'un couvercle 35 laissant passer de l'eau à travers des fentes 40 dont la largeur ne permet pas le passage des éléments inertes.

Une surface en pente 36 fait converger l'urine vers une zone de convergence 42 où se situent un moyen de capture 39 d'un volume prédéterminé d'urine de premier jet et un moyen 37 de prélèvement d'une partie de l'urine résiduelle. Opposé à la zone de convergence, par rapport au centre du bac 32, soit au point le plus haut de la ligne d'intersection des surfaces planes en pente, se situe une évacuation 38 qui permet de gérer le trop plein du bac et de vidanger les eaux de rinçage.

Ainsi, le dispositif de collecte d'urine 30 d'un animal prédéterminé comporte :
- un bac 32 de retenue d'éléments inertes vis-à-vis de l'urine, sur un tamis 35 configuré pour retenir les éléments inertes et laisser passer l'urine,
- une surface en pente 36, sous le tamis 34, pour rassembler l'urine en une zone de convergence 42,
- un moyen 39 de capture d'un volume prédéterminé d'urine de premier jet arrivant en premier dans la zone de convergence 42 et
- un moyen 37 de prélèvement d'une partie de l'urine résiduelle.

Ainsi, l'animal se sent comme sur une litière lorsqu'il urine dans le bac de retenue. De plus, l'urine prélevée provient d'au moins un jet postérieur au premier jet. L'urine est ainsi plus révélatrice des éventuels défauts de santé de l'animal. Le dispositif 30 est facile à nettoyer puisqu'il suffit de faire passer de l'eau dans les éléments inertes, sur le tamis, sur la pente et sur le moyen 39 de capture et le moyen 37 de prélèvement. Un propriétaire d'animal domestique peut ainsi procéder à un prélèvement d'urine sans l'aide d'un vétérinaire. Et ce prélèvement peut être de meilleure qualité que celle pratiquée par un vétérinaire car l'animal peut être à jeun, ne pas avoir fait d'efforts physiques et ne pas avoir bu.

Préférentiellement, la surface en pente 36 comporte, au moins en surface en contact avec l'urine, un matériau hydrophobe. L'écoulement de l'urine est ainsi facilité et la vitesse de convergence de l'urine est plus stable, ce qui favorise l'arrivée en premier, dans la zone de convergence, de l'urine du premier jet. Par exemple, la surface en pente hydrophobe comporte du PEHD grade 6.

Dans le mode de réalisation illustré en figures 1 à 5, la surface en pente 36 comporte deux pentes convergeant vers une ligne d'intersection inclinée vers la zone de convergence. Cette forme de la surface en pente favorise son nettoyage et la régularité de la vitesse d'écoulement de l'urine. Préférentiellement, la pente de la surface en pente 36 est comprise entre trois et cinq degrés d'angle. Cette pente permet l'arrivée de l'urine de premier jet en premier dans la zone de convergence.

Dans des modes de réalisation non représentés, la surface en pente présente une forme en accordéon, succession de surfaces sensiblement rectangulaires formant des canaux dont les coupes ont des formes en « V », les parties basses des canaux étant en pente vers une surface de collecte, perpendiculaire aux canaux, inclinée vers la zone de convergence. La vitesse d'écoulement de l'urine est ainsi régulière et le premier jet d'urine arrive en premier dans la zone de convergence.

Les éléments inertes comportent préférentiellement des granules plastiques sphériques ou cylindrique hydrophobes. L'urine est ainsi faiblement retenue par les éléments inertes et leur lavage est facilité par leur forme et leur qualité hydrophobe.

Par exemple, les granules sont des granules plastiques non absorbantes Purell (marque déposée) Grade 6 (médical) doc Purell ga7760 ou des granules plastiques PEHD USP classe 6 (fournisseur type ALBIS, marque déposée). Ces granules simulent une litière traditionnelle.

Le volume utile du moyen de capture est adapté en fonction de l'espèce animale prédéterminée (par exemple chien ou chat) afin de correspondre à un volume d'un premier jet d'urine relatif au volume de miction total de l'animal et présumé non exploitable pour l'analyse. Ce volume utile d'urine est préférentiellement maintenu piégé en fond de réceptacle pour être rendu inexploitable pour le reste de l'analyse. Le piégeage de ce volume utile est, par exemple un tampon d'acétate de cellulose ou autre matière absorbante, et/ou un système d'au moins une chicane mécanique maintenant le volume au fond du moyen de capture. Un deuxième volume surnageant appelé « volume tampon » est compris entre le volume rendu captif ou « volume de décantation » au fond du dispositif et le liquide biologique exploitable pour analyse, ou « volume de flottation ».

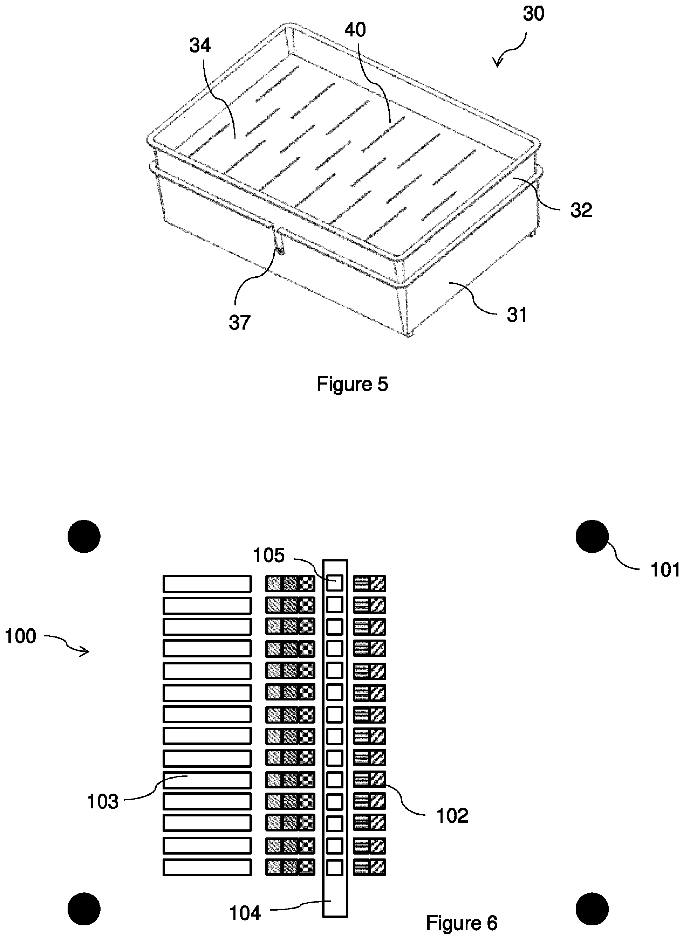

La bandelette peut être analysée par le propriétaire de l'animal, par exemple par comparaison avec des couleurs de témoin identifiant des quantités de composants dans l'urine résiduelle (voir le nuancier 100 illustré en, figure 6).

Préférentiellement, le récipient 70, 80, 85 ou 90 est, au moins partiellement, transparent dans les longueurs d'onde visibles. Ainsi, les caractéristiques physiques du contenu du récipient peuvent être observées, en particulier, la couleur de l'urine et la turbidité ou clairance de l'urine. Un récipient de prélèvement transparent permet de prendre en photo la clairance et la transparence de l'échantillon d'urine, données utiles au diagnostic biologique.

L'entrée 71, 81, 86 ou 97 du récipient 70, 80, 85 ou 90 est préférentiellement muni d'un bouchon.

En variante, le récipient 70, 80 ou 85 prend la forme externe d'un tube allongé, ou « stylo ».

L'intérêt du récipient 70, 80, 85 ou 90, pour un vétérinaire, est de pouvoir effectuer une lecture d'une bandelette, de contrôler la couleur et clairance d'une urine et de disposer d'un échantillon d'urine pour analyses complémentaires, sans avoir à reprélever l'animal.

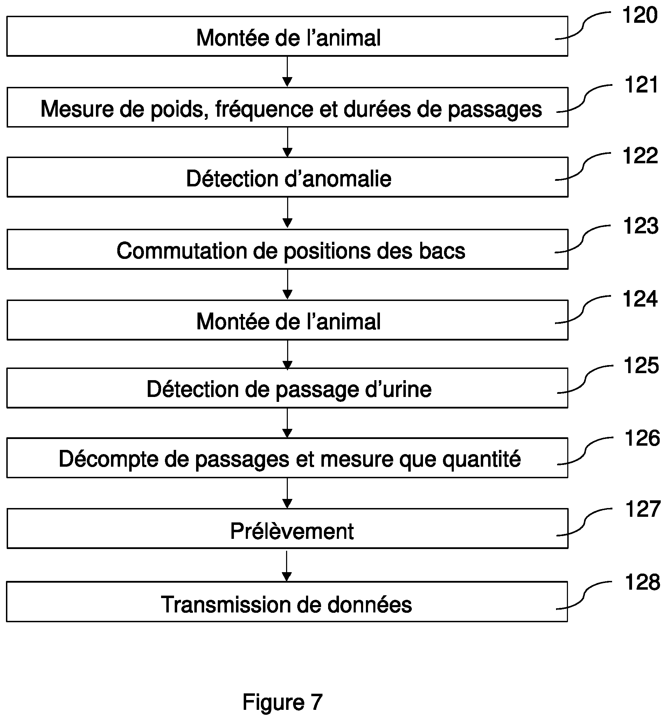

Pour la mise en œuvre du dispositif, la succession d'étapes illustrée en figure 7 peut être réalisée. Au début de cette succession d'étapes, le dispositif est dans sa configuration où la bac à litière 31 est au-dessus du bac de prélèvement d'urine 32. Au cours d'une étape 120, l'animal monte sur le bac 32. Au cours d'une étape 121, le dispositif mesure le poids de l'animal, par différence entre la mesure immédiate après stabilisation de l'animal et la mesure prise après la commutation de position de bacs. Au cours de cette même étape 121, le dispositif détermine la fréquence (le nombre sur une durée prédéterminée) et la durée de passage de l'animal sur la bac à litière 31. Le dispositif mémorise le poids, la fréquence et la durée ainsi mesurés.

Au cours d'une étape 122, le dispositif détermine une éventuelle anomalie dans les données mesurées, par comparaison avec des valeurs limites pour l'animal considéré. Le dispositif déclenche alors un message d'alerte à destination de l'application chargée sur le téléphone mobile du propriétaire de l'animal.

Au cours d'une étape 123, le propriétaire de l'animal commute les positions respectives de bacs pour que le bac 32 se trouve au-dessus du bac 31, comme illustré en figures 1 à 5 et retire le couvercle 34. Au cours d'une étape 124, l'animal monte sur le bac 32.

Au cours d'une étape 125, le dispositif détecte le passage d'urine, par exemple avec les composants illustrés en figure 6. Au cours d'une étape 126, le dispositif décompte le nombre de fois où de l'urine est passé pendant une durée prédéterminée, par exemple pour une demi-journée ou une journée, estime la quantité d'urine (en tenant compte d'une quantité restant retenue dans les granules et sur les surfaces de contact avec l'urine) et mémorise ces valeurs. Au cours d'une étape 127, l'utilisateur effectue un prélèvement d'urine résiduelle après capture d'urine de premier jet. Au cours d'une étape 128, les données mesurées sont transmises, par exemple à un téléphone mobile communicant ou à une box WiFi (passerelle domestique d'accès à internet). Lorsque le propriétaire juge qu'il n'est plus nécessaire d'effectuer des prélèvements d'urine, il positionne le couvercle 34 sur le bac 32, comme illustré en figures 1 à 5 et nettoie le bac 32 et les granules qu'il retient par passage d'eau claire. Puis il replace le bac 32 en dessous du bac 31.

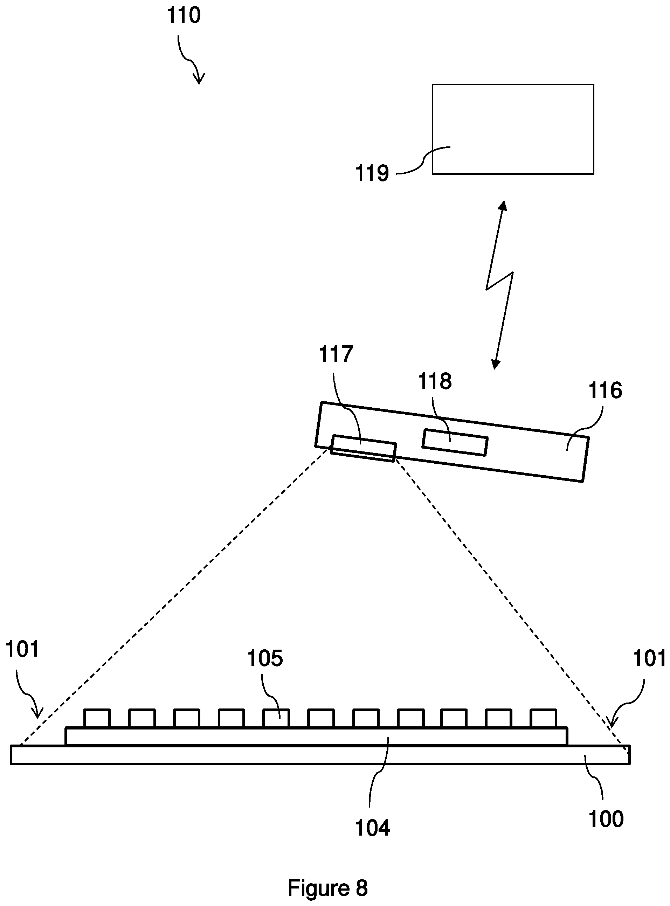

On observe, en figure 8, un moyen 110 d'analyse d'urine prélevée, qui comporte, sur un nuancier 100, par exemple similaire au nuancier illustré en figure 6, une bandelette 104 munie d'une pluralité de témoins 105 comportant, chacun, au moins un réactif. Chaque réactif réagit avec un composant particulier de l'urine. L'évolution de la couleur de chaque témoin plongé dans l'urine prélevée représente donc un taux du composant particulier dans l'urine. Le nuancier 100 comporte aussi des marques 101 destinée à aider la mesure de la déformation de l'image due au défaut de parallélisme d'un capteur d'image et du nuancier 100. Le nuancier 100 représente, en regard de chaque témoin 105, des couleurs 102 que les témoins prennent pour des taux prédéterminés de composants dans l'urine prélevée.

Avec un capteur d'image matriciel 117 muni d'une optique, par exemple celui d'un téléphone mobile 116, l'utilisateur prend une image de la bandelette 104 et du nuancier 100. Le téléphone mobile est configuré pour communiquer avec un serveur 119, par l'intermédiaire d'un réseau sans fil, par exemple un réseau WiFi ou un réseau de téléphonie mobile. Soit un processeur 118 du téléphone mobile 116, soit le serveur 119 effectue les traitements d'image exposés ci-dessous pour déterminer les taux de composants de l'urine qui sont révélés par les réactifs des témoins 105.

Ainsi, avec un simple téléphone mobile, le propriétaire de l'animal peut procéder à une première analyse de l'urine pour obtenir simultanément une évaluation des quantités de plusieurs composants dans l'urine prélevée et, en fonction des résultats de cette analyse, décider de consulter un vétérinaire.

Préférentiellement, le processeur du téléphone mobile 116 ou le serveur 119 est aussi configuré pour traiter une image de l'urine présente dans le récipient ou boîtier 70, 80, 85 ou 90 afin de déterminer la couleur de l'urine ainsi que sa clairance.

Préférentiellement, le téléphone mobile 116 ou le serveur 119 comporte un moyen de compensation des variations d'éclairage de chaque témoin. Par exemple, le moyen d'analyse 110 comporte un moyen de comparaison de la couleur de chaque témoin 105 avec les couleurs 102 du nuancier 100 qui sont en regard de chaque témoin. Alternativement, téléphone mobile 116 ou le serveur 119 réalise une balance des blancs sur le fond blanc du nuancier 100. Ainsi, quelles que soient les variations de l'éclairage, naturel ou artificiel, des témoins, la couleur de chaque témoin est évaluée après compensation de ces variations.

Préférentiellement, téléphone mobile 116 ou le serveur 119 comporte un moyen de comparaison :
- d'au moins une couleur évaluée avec au moins une valeur limite de couleur, les valeurs limites de couleur étant représentées par les couleurs 102 et/ou
- d'au moins une quantité évaluée de composant de l'urine avec au moins une valeur limite de quantité.

Le propriétaire de l'animal peut être automatiquement prévenue d'une possible anomalie de la composition de l'urine et d'un potentiel problème de santé de l'animal. Toutes ces données sont partagées avec le vétérinaire ; il peut alors décider de recevoir l'animal en consultation et continuer le traitement mis en place. Le propriétaire peut, de son côté, décider de consulter un vétérinaire sur la base d'éléments objectifs.

La communication entre le téléphone mobile 116 et le serveur 119 comporte l'émission de couleurs évaluées et/ou de résultats de comparaison des couleurs évaluées. Préférentiellement, le téléphone mobile 116 comporte un moyen de réception depuis le serveur 119 distant, de valeurs limites de couleur et/ou de quantités de composant dans l'urine. Ainsi, le praticien ou le vétérinaire, peut recevoir et/ou paramétrer l'analyse d'urine effectuée par le moyen d'analyse.

La communication à distance entre moyen d'analyse 110 et le serveur 119 permet au praticien d'effectuer :
- un suivi patient pour comparatif spécifique à l'animal (autoréférence)
- un enrichissement et comparatif d'une base de données biologiques (pour améliorer la détection des symptômes, les diagnostics et les effets des traitements)

Les différences étapes nécessaires pour l'extraction de la zone du nuancier qui correspond à la couleur d'un témoin, après acquisition d'une image numérique comportent :
- la recherche des marques 101,
- des tests sur la distance de prise de vue entre le nuancier 100 et le téléphone mobile 116,
- des tests sur la perspective, angle entre le nuancier et le smartphone,
- le calcul de l'angle de rotation du nuancier par rapport au capteur d'image 117,
- une extraction de la zone de l'image où se situent les couleurs du nuancier,
- un alignement horizontal et vertical par rotation de la grille du nuancier,
- un calcul de la déformation perspective de l'image et
- une correction des défauts de perspective.

Tous ces étapes permettent d'optimiser l'acquisition d'images.

La communication entre le téléphone mobile 116 et le serveur 119 comporte une identification du propriétaire et de l'animal.

Trois différents algorithmes de comparaison des couleurs peuvent notamment être utilisés pour la reconnaissance des couleurs des pastilles d'une bandelette urinaire en comparaison avec un nuancier.

Ces trois algorithmes sont la corrélation d'histogrammes dans l'espace HSV (en anglais Hue Saturation Value ou TSV en français pour Teinte Saturation Valeur), une méthode de mesure de distance HSV et une méthode de mesure de distance LAB.

La corrélation d'histogrammes dans l'espace HSV utilise les canaux H et S de l'image convertie au format HSV. On part ainsi du principe que le canal V, étant entaché par les variations d'éclairage, risque de fausser les résultats. Le but de l'algorithme consiste à calculer la corrélation entre les histogrammes H et S provenant de chaque couleur 102 du nuancier et du témoin 105 correspondant de la bandelette.

Après avoir défini le nombre de classes à prendre en compte dans chaque histogramme, H et S, on exécute les étape suivantes :
pour chaque ligne du nuancier :
   - calcul des histogrammes H et S pour le témoin 105 de la bandelette 104 situé sur cette ligne ;
   - normalisation de ces histogrammes ;
pour chaque couleur de référence 102 du nuancier sur cette ligne :
   - calcul des histogrammes H et S pour cette couleur de référence ;
   - normalisation de ces histogrammes ;
   - calcul du coefficient de corrélation entre les histogrammes des couleurs de référence 102 et la couleur du témoin 105.

Puis, pour chaque ligne, on recherche le coefficient de corrélation maximal, pris en compte uniquement si la séparation entre les deux coefficients les plus grands est supérieure à une valeur limite prédéterminée (test de pertinence).

Enfin, on effectue un test de validité : on estime que les coefficients de corrélation sont valables si leur valeur dépasse une valeur limite prédéfinie. En général, en statistiques, cette valeur limite vaut 0,8. Toutefois, une valeur limite aussi faible que 0,1 indique déjà une reconnaissance correcte de la couleur.

En ce qui concerne l'algorithme de mesure de la distance HSV, l'espace HSV étant de forme cylindrique, pour y calculer une distance euclidienne, il est nécessaire de projeter les valeurs H, S, V sur les axes principaux. Dans un deuxième temps, on évalue la similarité entre une couleur référence le la couleur du témoin, en calculant le carré de la distance euclidienne pondérée qui les sépare.

Un coefficient de normalisation dépend des poids affectés à chacun des canaux H, S et V.

Généralement, on peut affecter au poids du canal V une valeur très faible, voire nulle, afin de s'affranchir des variations de l'éclairage.

Puis, pour chaque ligne, on recherche la similarité maximale. Enfin, on effectue un test de validité : on estime que les similarités sont valables si leur valeur dépasse une valeur limite prédéfinie.

Dans le cas de l'algorithme de mesure de la distance LAB, l'espace LAB étant linéaire par nature, la distance euclidienne pondérée se calcule directement. Dans une deuxième phase, pour chaque ligne, on recherche la distance la plus petite.

La base de données conservée par le serveur 119 permet d'établir un prédiagnostic de l'état de santé de l'animal en fonction des résultats obtenus par l'intermédiaire de la capture et du traitement d'image ainsi que des informations sur les caractéristiques de l'animal comme le poids, l'âge, l'espèce, la race et le sexe.

Les diagnostics finaux établis par les vétérinaires sont stockés dans la base permettant son enrichissement au fur et à mesure que des cas sont enregistrés dans le but d'affiner les prédiagnostics initiaux.

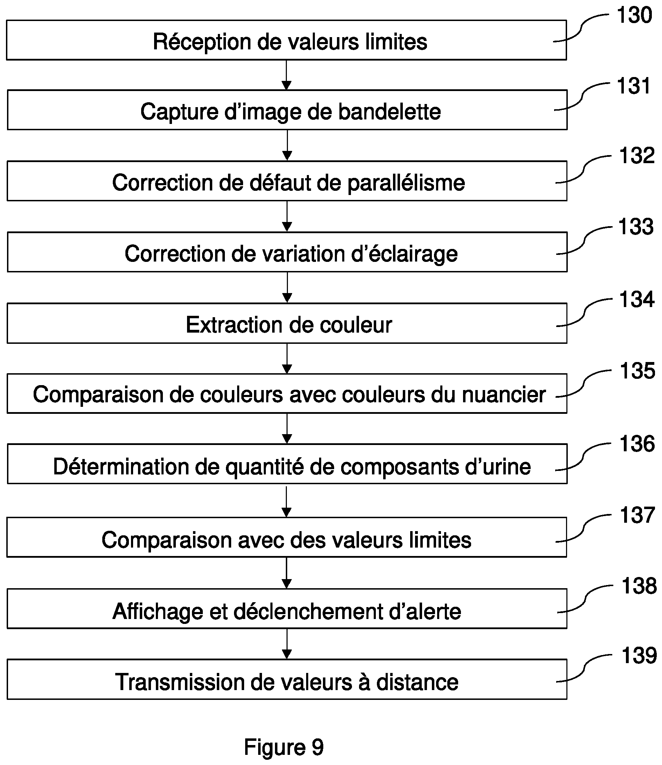

La figure 9 représente, sous forme de logigramme, des étapes d'analyse de prélèvement d'urine, réalisées par une application chargée sur un téléphone mobile 116. Au cours d'une étape 130, l'application reçoit, depuis le serveur 119, des valeurs limites à appliquer pour l'animal considéré. Au cours d'une étape 131, le téléphone mobile 116 effectue une capture d'image de la bandelette. Au cours d'une étape 132, l'application réalise la correction des défauts de parallélisme, en appliquant une déformation à l'image captée qui met restitue une image dans laquelle, les marques 101 forment les angles d'un rectangle.

Au cours d'une étape 133, l'application réalise une correction de variation d'éclairage. Au cours d'une étape 134, l'application réalise une extraction de couleur de chaque témoin. Au cours d'une étape 135, l'application réalise une comparaison de la couleur du témoin avec les couleurs du nuancier qui se trouvent en regard (c'est-à-dire sur la même ligne) du témoin considéré. On note que les étapes 133 à 135 peuvent être rassemblées en une seule étape lorsque l'on effectue des corrélations comme exposé plus haut. Au cours d'une étape 136, l'application ou le serveur 119 détermine les quantités ou taux des composants correspondant aux réactifs des témoins dans l'urine prélevée. Au cours d'une étape 137, l'application ou le serveur 119 compare les valeurs de quantité ou de taux déterminées au cours de l'étape 136 avec les valeurs limites reçues au cours de l'étape 130. Au cours d'une étape 138, l'application provoque l'affichage, sur l'écran du téléphone mobile 116, des résultats obtenus et, en cas de dépassement des valeurs limites reçues, d'un message d'alerte invitant le propriétaire à consulter le vétérinaire. Au cours d'une étape 139, si l'application a réalisé les étapes 136 et 137, l'application transmet les résultats obtenus au serveur 119.

Bien entendu, la répartition des étapes entre l'application et le serveur 119 peut être modifiée, dans des variantes non explicitées.

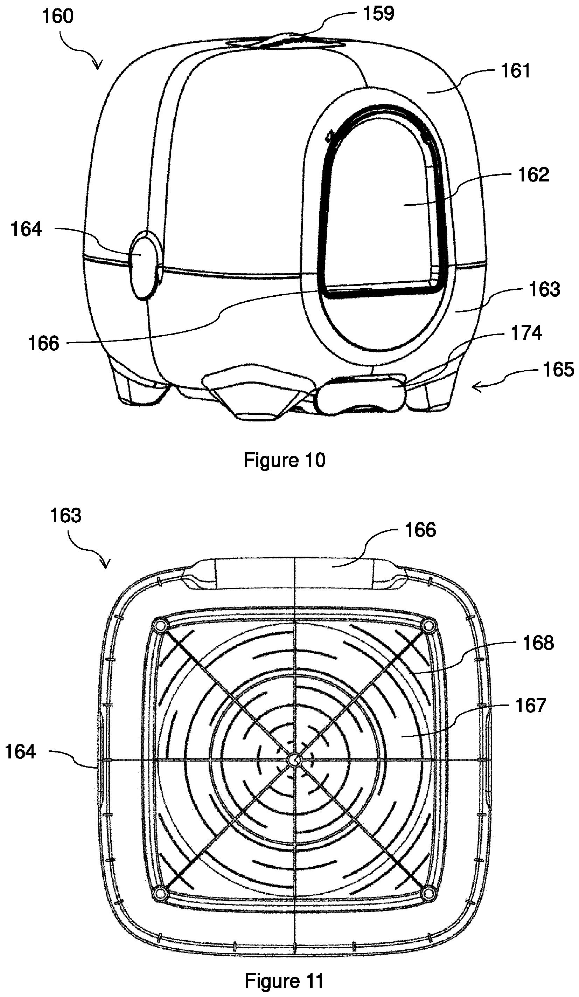

Les figures 10 à 20 représentent un mode de réalisation particulier 160 du dispositif objet de l'invention.

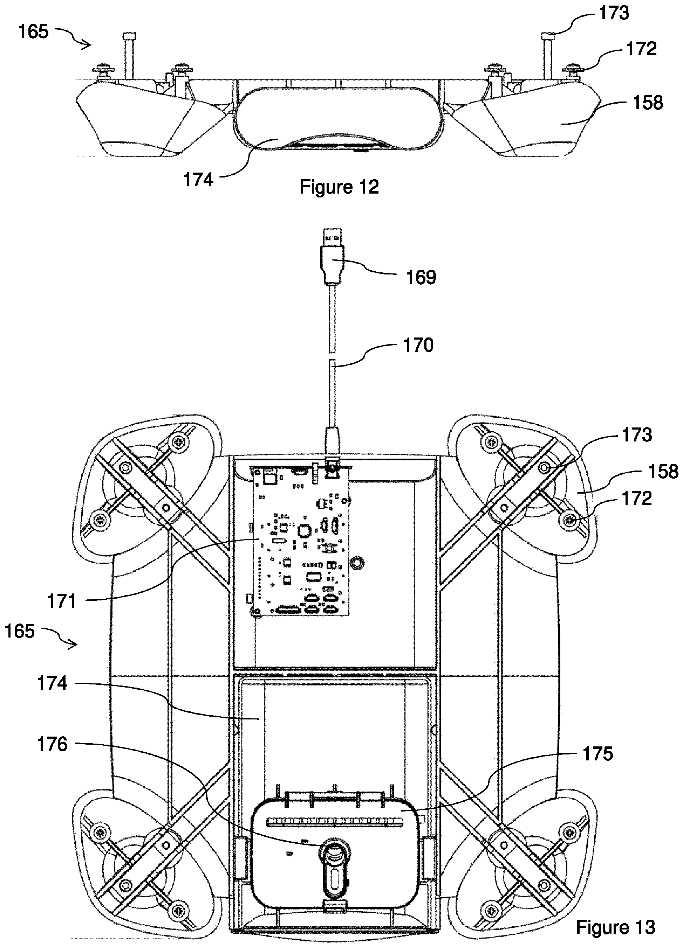

Comme illustré en figure 10, le dispositif 160 comporte un demi-boîtier supérieur 161 muni d'une poignée 159 et d'une porte basculante 162, et un demi-boîtier inférieur 163 muni d'une assise 165, d'un passage 166 et d'un tiroir 174. Les deux demi-boîtiers 161 et 163 sont reliés par des attaches 164. Lorsque l'animal va uriner, il entre dans le dispositif 160 en poussant la porte 162 pour la faire basculer. Lorsque le propriétaire veut changer la litière ou laver les composants du dispositif, il déverrouille les attaches 164, soulève le demi-boîtier supérieur 161 en utilisant la poignée 159. Il accès ainsi aux bacs superposés. La figure 11 illustre le demi-boîtier inférieur 163 une fois retiré le demi-boîtier supérieur 161 lorsque le bac à litière a été retiré ou qu'il se trouve en dessous du bac de retenue d'éléments inertes. Dans cette figure 11, on observe le bac 167 d'accueil de l'animal urinant et de retenue d'éléments inertes vis-à-vis de l'urine, sur un tamis dont les ouvertures 168 en segments de cercles concentriques sont représentées. Comme détaillé en regard du premier mode de réalisation du dispositif, le tamis 168 est configuré pour retenir des éléments inertes vis-à-vis de l'urine et pour laisser passer l'urine de l'animal. Une surface en pente (non représentée), sous le tamis 168, rassemble, par gravité, l'urine en une zone de convergence (non représentée), depuis laquelle l'urine s'écoule dans un boîtier 175 de collecte et d'analyse d'urine (voir figures 13 à 20).

Comme illustré en figures 12 et 13, l'assise 165 comporte quatre pieds 158 munis de plots de guidage 173 et d'appuis 172 de mesure de poids. Les plots de guidage évitent que les mouvements de l'animal décale le demi-boîtier inférieur 163 de l'assise 165. Les appuis 172 reçoivent, conjointement, tous le poids des éléments du dispositif hormis l'assise 175. Comme détaillé ci-dessus, par comparaison entre les poids mesurés sans l'animal, avec l'animal puis sans l'animal mais avec l'urine, le dispositif détermine le poids de l'animal et son évolution et la quantité d'urine libérée par l'animal. L'assise 165 comporte une carte électronique 171 munie d'un connecteur pour relier un câble 170 muni d'un deuxième connecteur 169, par exemple mettant en œuvre les spécification USB (acronyme de Universal Serial Bus pour bus série universel).

La carte électronique 171 comporte un composant programmable, par exemple un microcontrôleur, et une mémoire de programme de traitement de données, aussi appelé, par la suite « algorithme ».

L'assise comporte aussi le tiroir 174 qui retient en position le boîtier amovible 175. L'ouverture de pénétration de l'urine dans le boîtier 175 est représentée en 176.

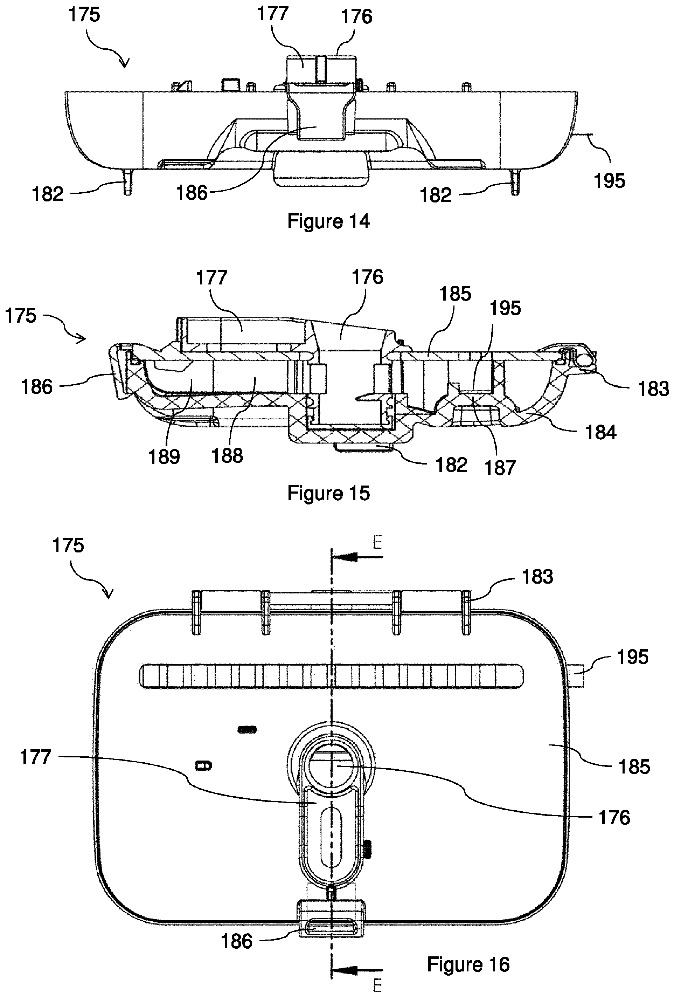

Le boîtier 175 est représenté, plus en détail, en figures 14 à 20. Le boîtier 175 présente des dimensions d'environ 120 mm sur 80 mm (vues de dessus). Le boîtier conserve un échantillon de qualité de deuxième jet disponible, conservé dans un grade médical / alimentaire.

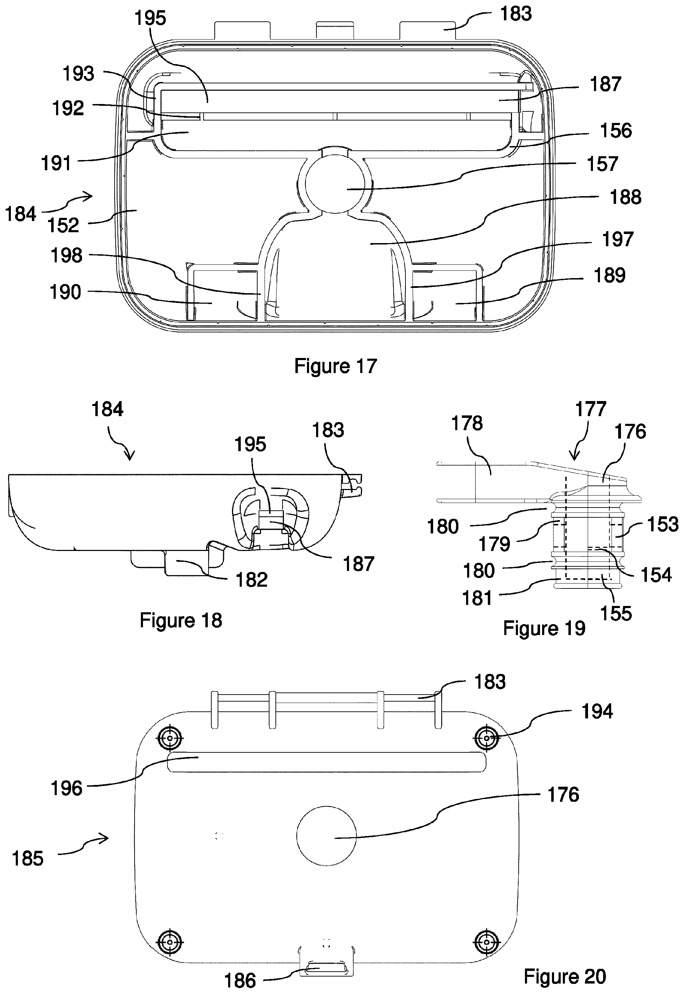

Comme on l'observe dans ces figures, le boîtier 175 comporte trois éléments mobiles entre eux, une base 184 (voir figures 17 et 18), un couvercle 185 (voir figure 20) et une bride 177 (voir figure 19). Le couvercle 185 est monté sur une charnière 183 de la base 184. Le couvercle 185 est verrouillé sur la base 184 par une attache flexible en clip 186. La bride 177 est mobile en rotation autour d'un axe, vertical en figures 14, 15, 18 et 19. Les pieds 182 matérialise au moins un axe de basculement du boîtier 175. Dans le mode de réalisation illustré dans les figures, les pieds 182 ont une forme plane globalement en rectangle, l'axe de basculement passant d'un sommet inférieur du rectangle à un autre au cours du basculement. Dans des variantes, les pieds ont une forme plane globalement en triangle, dont un sommet est orienté vers le bas et correspond à l'axe de basculement. Dans des variantes, les pieds ont une forme plane globalement en cercle, l'axe de basculement coulissant le long du cercle au cours du basculement.

La base 184 comporte un support 187 de bandelette 195 formant guide en translation. La base 184 comporte un premier réceptacle d'urine 188 comportant des compartiments latéraux 189 et 190. La base 184 comporte aussi un deuxième réceptacle 191 isolé du premier réceptacle 188 par une cloison 156 munie d'un passage 157. Le support 187 et guide de bandelette 195 comporte une butée 193 et des picots 192.

Comme illustré en Figure 19, la bride 177 comporte une poignée 178, un bouchon inférieur 181, deux emplacements de joints toriques 180, une canalisation interne verticale dont la partie supérieure est l'entrée d'urine 176, une canalisation horizontale 153. Le bouchon 181 comporte un réservoir d'urine de premier jet 155 et une cloison horizontale 154 de retenue d'urine dans le réservoir 181. Lorsque l'urine pénètre dans le boîtier 175, par l'entrée 176, le boîtier 175 est dans une première configuration dans laquelle l'urine est orientée, par gravité vers le premier réceptacle 188. Par rapport à la configuration intermédiaire illustrée en figure 15, dans la première configuration, le boîtier 175 a tourné dans le sens trigonométrique et est en appui sur ses pieds 182 et sur l'arête du boîtier se trouvant sous le premier réceptacle 188. Dans cette première configuration, l'urine commence par remplir de réservoir d'urine de premier jet 155, puis le premier réceptacle. Lorsque l'animal a fini d'uriner, l'utilisateur extrait le boîtier 175 du dispositif 160 en ouvrant le tiroir 174. Puis, il fait basculer le boîtier 175 dans une deuxième configuration dans laquelle l'urine présente dans le premier réceptacle 188 qui n'est pas retenue dans l'un des compartiments 189 et 190 traverse la vanne 177 pour atteindre le deuxième réceptacle 191, par gravité. Par rapport à la configuration intermédiaire illustrée en figure 15, dans la deuxième configuration, le boîtier 175 a tourné dans le sens horaire et est en appui sur ses pieds 182 et sur l'arête du boîtier se trouvant sous le deuxième réceptacle 191. On observe que l'urine présente dans le réservoir 155 y est retenue par la cloison 154.

En variante, le réservoir 155, constituant un moyen de capture de premier jet d'urine comporte une matière absorbante configurée pour absorber l'urine, en complément ou à la place de la cloison 154.

Après avoir positionné le boîtier 175 dans la deuxième configuration, l'utilisateur ferme la vanne multivoie 177 en la faisant tourner, par rapport à son axe, d'un quart de tour. Il effectue ensuite une capture d'image, environ une minute après l'imprégnation de la bandelette 195, du couvercle 185 et, à travers ses fenêtres, de la bandelette 195 et de l'urine présente dans les compartiments 189 et 190.

Le couvercle 185 est relié à la base 184 par un joint d'étanchéité. Le premier réservoir 155 présente un volume de l'ordre de 10 à 15 pourcents du volume de la miction. Le premier réceptacle 188 présente un volume de l'ordre de quatre millilitre, préférentiellement entre trois et six millilitres. Chacun des compartiments 189 et 190 présente un volume de l'ordre d'un millilitre. Le volume aliquote 152 entourant le premier réceptacle 188 recueille, par débordement, de l'urine. Il présente un volume, par exemple de 15 à 25 millilitres.

Comme illustré en figure 20, le couvercle 185 comporte des repères 194 facilitant le repérage dans une image et une fenêtre 196 d'observation de la bandelette 195. Le couvercle 185 est transparent pour permettre la mesure de la couleur dans le compartiment 190 et de la turbidité dans le compartiment 189.

L'urine présente dans le premier réceptacle 188 pénètre dans compartiments 189 et 190 par débordement au dessus de cloisons 197 et 198 et/ou par passage dans des chicanes représentées dans en figure 17. Le couvercle présente préférentiellement au moins un réglette de niveau d'urine dans chacun des réceptacles 188 et 191 et des compartiments 189 et 190.

Toutes les caractéristiques particulières de l'un des modes de réalisation du boîtier ou récipient sont compatibles avec celles d'un autre mode de réalisation du boîtier ou récipient pour former des variantes de ces modes de réalisation. Toutes les caractéristiques particulières de l'un des modes de réalisation du dispositif sont compatibles avec celles de l'autre mode de réalisation du dispositif pour former des variantes de ces modes de réalisation du dispositif objet de l'invention.

Un mode d'utilisation du dispositif objet de l'invention est décrit ci-après. Suite à l'acquisition d'un dispositif, le vétérinaire configure le profil de l'animal afin de mettre en place un premier niveau de valeurs limites (seuillage) personnalisé en fonction de la race, du sexe, de l'âge, du poids et des antécédents médicaux de l'animal. Ces premières valeurs limites (« seuils ») concernent notamment le poids idéal de l'animal ainsi que le comportement dit « normal » mais également des niveaux de concentrations déjà constatés comme le taux de glucose dans les urines généralement constaté lors de tests antérieurs. Après une durée déterminée d'utilisation du dispositif au domicile de l'animal, un second niveau de seuillage est positionné automatiquement en fonction des données récoltées et traitées par les algorithmes. Ces seuils concernent les habitudes quotidiennes de l'animal, le nombre de passages quotidien, la durée des passages dans le dispositif afin de détecter au plus tôt toute déviance du comportement pouvant être assimilé à des premiers symptômes d'une maladie. Des dépassements des valeurs limites (« seuils ») alertent le vétérinaire (et/ou le propriétaire) afin qu'il puisse décider du besoin de réaliser un test urinaire via le boîtier 175. Les résultats observés sur le test urinaire de l'animal permettent au vétérinaire d'affiner les seuils de contrôle pour les prochains tests et de décider si une visite médicale de l'animal est nécessaire.

Lors de la récolte de l'urine, la position d'attente du boîtier 175 est inclinée dans une première configuration dans laquelle l'urine s'écoule vers le premier réceptacle 188. Simultanément, la vanne 177 est dans la configuration représentée en figures 14 à 16 permettant la pénétration de l'urine dans le premier réceptacle.

Concernant l'acquisition de l'image, l'algorithme contrôle le positionnement du capteur d'image (généralement celui d'un ordiphone, « smartphone », d'une tablette ou d'une caméra internet, « webcam ») par rapport à la bandelette et son support, mesure la luminosité puis prend une image de l'ensemble du couvercle du boîtier ou du boîtier ouvert (cas non détaillé ci-dessous). Les paramètres de prise de vue que l'on peut faire varier se répartissent en deux catégories : les paramètres externes au capteur d'image et les paramètres internes. Les contrôles externes comportent :
- l'éclairage : uniformité, scintillement, intensité, disposition par rapport au capteur,
- la distance de prise de vue,
- les angles de prise de vue : angle latéral et angle de plongée et
- la position de la bandelette au sein du dispositif.

Les contrôles internes comportent :
- la balance des blancs et
- la focalisation (autofocus).

Concernant le traitement de l'image, l'algorithme redresse l'image (correction d'angles) afin de pouvoir identifier et cloisonner les différents éléments qui composent l'image du couvercle. On vient ainsi déterminer différentes parties et sous-parties essentielles à la collecte des données. L'algorithme identifie la zone où est située la bandelette urinaire et cloisonne ensuite les 15 pastilles qui la composent, déterminant ainsi 15 zones distinctes (14 pastilles « analytes » et une pastille de référence). Les 14 analytes concernent, par exemple, les leucocytes, les corps cétoniques, le nitrite, l'urobilinogène, la bilirubine, le glucose, les protéines, la densité, le potentiel hydrogène (pH), la présence de sang, l'acide ascorbique, la micro-albumine, la calcium et la créatinine. Deux rapports sont calculés : RPCU, Protéine / Créat urinaire et MACU, microalbumine / créat urinaire.

L'algorithme identifie aussi la zone des cuves urinaires et la cloisonne en sous-parties : cuve 188 (Volume « utile »), cuve 190 (Couleur de l'urine) et cuve 189 (Turbidité de l'urine). L'algorithme vient ensuite analyser les différentes sous-parties de l'image afin de convertir ces images en données numériques. Les couleurs des pastilles de la bandelette sont alors comparées avec celle de nuanciers de couleurs de références préalablement numérisés afin d'identifier les valeurs numériques associées.

Trois différents algorithmes de comparaison des couleurs ont été sélectionnés. Ces trois algorithmes sont la corrélation d'histogrammes dans l'espace HSV (en anglais Hue Saturation Value ou TSV en français pour Teinte Saturation Valeur), une méthode de mesure de distance HSV et une méthode de mesure de distance LAB. L'algorithme fait un second niveau de contrôle du niveau d'éclairage sur la pastille de référence et ajuste en fonction la variable luminosité pour éviter les faux positifs. La couleur de l'urine est déterminée en comparaison avec un nuancier de couleurs d'urines animales suivant la même méthodologie que pour déterminer la couleur des pastilles de la bandelette. L'algorithme détermine quelle est la couleur du nuancier qui correspond le mieux à la couleur observée sur l'image. L'algorithme de mesure de turbidité reprend le test sur la turbidité qui est utilisé par les praticiens, c'est à dire qu'un texte est positionné de manière lisible dans la Cuve Cb, en présence d'un liquide, si le texte n'est plus lisible, l'échantillon sera considéré comme étant trouble et différents niveaux de turbidité peuvent être indiqué en fonction de la lisibilité de ce texte. L'algorithme indique que l'urine est trouble si le texte n'est pas visible ou clair si le texte est parfaitement visible, d'autres niveaux intermédiaires pouvant également être déterminés. L'algorithme recalcule aussi le niveau d'urine présent dans le boîtier en additionnant les niveaux calculés dans chacun des différents compartiment 188, 189, 190, 191.

Concernant le contrôle du comportement du chat (heure, durée et fréquence de passage), au-delà d'un profil standard d'un chat, on caractérise l'animal par l'observation de ses propres habitudes sur les premières semaines (analyses du comportement) et l'on attribue des valeurs aux paramètres de son profil (seuils personnalisés). Tout dépassement significatif (c'est-à-dire au-delà d'une tolérance prédéterminée) de ces seuils renseigne automatiquement le propriétaire et le vétérinaire de toutes anomalies pouvant justifier une consultation du vétérinaire.

Les seuils du poids de forme est fixé par le vétérinaire suivant la race, âge, sexe, et à la suite d'une consultation. Tout dépassement significatif de ces seuils renseigne automatiquement le propriétaire et le vétérinaire de toutes anomalies pouvant justifier une consultation du vétérinaire.

Compte-tenu du profil de l'animal et des seuillages « pathologiques » fixés par le vétérinaire, une analyse d'urine est proposée au propriétaire
Au-delà d'un profil standard d'un animal, le vétérinaire incrémente des seuils de 14 analytes permettant de renseigner des pathologies éventuelles comme le diabète, l'insuffisance rénale, les pbl Hépatiques, ...

L'interprétation des différentes anomalies ou suspicions permet de demander au propriétaire de prendre un rendez-vous en consultation et de conserver un échantillon d'urine prélevé. Le propriétaire emporte alors le boîtier 175 à cette consultation, si elle a lieu rapidement, par exemple le jour même.

Suivant la pathologie et son stade d'évolution, il est préconisé une fréquence d'analyses représentatives pour un diagnostic vétérinaire (par exemple, un suivi quotidien pour un diabète ou un suivi lors d'un traitement ou intervention).

Bien entendu, l'algorithme peut mettre en œuvre une intelligence artificielle qui va permettre par l'incrémentation de données (symptômes, comportements, résultats d'analyses suivant les profils de race, âges, sexe, ...) d'anticiper des pathologies, préconiser des conditions de vie (alimentaires, exercice, ...) pour que l'animal vive mieux et plus longtemps.

## Revendications

1. Boîtier (175) de capture et d'analyse d'urine d'un animal, qui comporte :
- un premier réceptacle d'urine (188) et
- un deuxième réceptacle d'urine (191) muni d'un support (187) de bandelette (195) portant des témoins comportant des réactifs, lesdits réceptacles étant séparés par une cloison (156) munie d'un passage (157), et au moins un support (182) matérialisant un axe de basculement du boitier entre :
- une première configuration dans laquelle, lorsque le passage est ouvert, l'urine s'écoule par gravité vers le premier réceptacle et
- une deuxième configuration dans laquelle, lorsque le passage est ouvert, l'urine s'écoule par gravité, vers le deuxième réceptacle ; et
- une vanne (177) qui obture ou ouvre le passage entre lesdits réceptacles
boitier dans lequel :
- le premier réceptacle (188) comporte au moins un compartiment latéral (189, 190) et au moins une cloison (197, 198) de retenue d'urine dans un compartiment latéral lorsque le boîtier bascule dans la deuxième configuration et/ou
- le dispositif comporte un couvercle (185) transparent au moins en regard du support (187) de bandelette (195), le couvercle étant mobile par rapport à une base (184) comportant les réceptacles (188, 191).

2. Boîtier (175) selon la revendication 1, dans lequel la vanne (177) est une vanne multivoie qui obture ou ouvre, de plus, une entrée (176) d'urine dans le boîtier.

3. Boîtier (175) selon la revendication 2, dans lequel la vanne (177) comporte un réservoir (155) de retenue du premier jet d'urine pénétrant dans le boîtier.

4. Boîtier (175) selon la revendication 3, dans lequel le réservoir (155) de retenue du premier jet d'urine est surmonté d'une cloison (154) limitant l'écoulement de l'urine vers le deuxième réceptacle (191), dans la deuxième configuration du boîtier.

5. Dispositif (160) de collecte d'urine d'un animal prédéterminé, qui comporte :
- un boîtier (175) de capture et d'analyse d'urine selon l'une des revendications 1 à 4,
- un bac (167) d'accueil de l'animal urinant et de retenue d'éléments inertes vis-à-vis de l'urine, sur un tamis (168) configuré pour retenir les éléments inertes et laisser passer l'urine,
- une surface en pente, sous le tamis, pour rassembler l'urine en une zone de convergence menant à une entrée du boîtier de capture et d'analyse d'urine.

6. Dispositif (160) selon la revendication 5, dans lequel le boîtier (175) est au moins partiellement transparent et comporte un réservoir d'urine prélevée (155) et un bouchon (181) d'accès à ce réservoir.

7. Dispositif (160) selon l'une des revendications 5 ou 6, qui comporte, de plus, un moyen d'analyse (100, 110, 116, 119) de l'urine prélevée.

8. Dispositif (160) selon la revendication 7, dans lequel le moyen d'analyse (100, 110 116, 119) comporte un capteur de lumière (117) pour capter la couleur d'au moins un témoin comportant au moins un réactif et un moyen (118, 119) de compensation des variations d'éclairage de chaque témoin.

9. Dispositif (160) selon la revendication 8, dans lequel le moyen d'analyse (100, 110, 116, 119) comporte un moyen (118, 119) d'évaluation de la couleur de l'urine prélevée et/ou un moyen d'évaluation de la clairance de l'urine prélevée.

10. Procédé d'utilisation d'un boîtier (175) selon l'une des revendications 1 à 4 ou un dispositif (160) selon l'une des revendications 5 à 9, qui comporte :
- une étape (131) de prise d'une image du boîtier,
- une étape d'identification d'une bandelette dans l'image captée,
- une étape (134) de détermination de couleur de pastilles portées par la bandelette,
- une étape (135 à 137) de comparaison d'au moins une couleur de pastille avec au moins une valeur limite de couleur, pour détecter une anomalie,
- une étape d'identification d'au moins un compartiment retenant de l'urine, dans l'image captée,
- une étape de mesure de turbidité et/ou de couleur de l'urine retenue dans au moins un compartiment et
- une étape de comparaison de la turbidité mesurée et/ou de la couleur mesurée avec au moins une valeur limite pour détecter une anomalie et
- une étape d'affichage de chaque anomalie détectée.

11. Procédé selon la revendication 10, qui comporte, de plus :
- une étape (130) d'initialisation de valeurs limites de détection d'anomalies et
- une étape de réévaluation d'au moins une valeur limite en fonction des valeurs mesurées pour l'animal considéré.

12. Procédé selon l'une des revendications 10 ou 11, qui comporte, de plus :
- une première étape de détermination de comportement de l'animal vis-à-vis du boîtier, par exemple heure, durée et fréquence de passage,
- une étape d'initialisation de valeurs limites relatives au comportement de l'animal,
- une deuxième étape de mesures de valeurs représentatives de comportement de l'animal et
- une étape de comparaison des mesures avec les valeurs limites, pour détecter une anomalie.

13. Procédé selon l'une des revendications 10 à 12, qui comporte, de plus :
- une première étape de détermination de poids de l'animal et/ou de poids d'au moins une miction de l'animal,
- une étape d'initialisation de valeurs limites relatives au poids déterminé,
- une deuxième étape de mesures de poids de l'animal et/ou de poids d'au moins une miction de l'animal et
- une étape de comparaison des mesures avec les valeurs limite, pour détecter une anomalie.

14. Procédé selon l'une des revendications 10 à 13, qui comporte, de plus, une étape de capture de de la couleur d'au moins un témoin comportant au moins un réactif et une étape (133) de compensation des variations d'éclairage de chaque témoin.

## Patentansprüche

1. Behälter (175) zum Auffangen und Analysieren von Tierurin, der Folgendes umfasst:
- einen ersten Urinbehälter (188) und
- einen zweiten Urinbehälter (191), der mit einer Halterung (187) für einen Teststreifen (195) ausgestattet ist, der Indikatoren mit Reagenzien trägt, wobei die Behälter durch eine Trennwand (156) mit einem Durchgang (157) voneinander getrennt sind, und mindestens einen Träger (182), der eine Kippachse des Behälters bildet zwischen:
- einer ersten Konfiguration, in der bei geöffnetem Durchgang der Urin durch Schwerkraft in den ersten Behälter fließt, und
- einer zweiten Konfiguration, in der bei geöffnetem Durchgang der Urin durch Schwerkraft in den zweiten Behälter fließt; und
- ein Ventil (177), das den Durchgang zwischen den genannten Behältern verschließt oder öffnet Behälter, bei dem:
- der erste Urinbehälter (188) mindestens ein seitliches Fach (189, 190) und mindestens eine Trennwand (197, 198) zum Zurückhalten von Urin in einem seitlichen Fach aufweist, wenn der Behälter in die zweite Konfiguration kippt, und/oder
- die Vorrichtung einen Deckel (185) aufweist, der zumindest gegenüber der Halterung (187) für den Teststreifen (195) transparent ist, wobei der Deckel relativ zu einer Basis (184) beweglich ist, die die Urinbehälter (188, 191) aufweist.

2. Behälter (175) nach Anspruch 1, wobei das Ventil (177) ein Mehrwegeventil ist, das zusätzlich einen Urin-Einlass (176) in den Behälter verschließt oder öffnet.

3. Behälter (175) nach Anspruch 2, wobei das Ventil (177) einen Tank (155) zum Auffangen des ersten in den Behälter eindringenden Urinstrahls umfasst.

4. Behälter (175) nach Anspruch 3, wobei der Tank (155) zum Auffangen des ersten Urinstrahls in der zweiten Konfiguration des Behälters mit einer Trennwand (154) versehen ist, die den Urinfluss zum zweiten Auffangbehälter (191) begrenzt.

5. Vorrichtung (160) zum Sammeln von Urin eines vorbestimmten Tieres, die Folgendes umfasst:
- ein Behälter (175) zum Auffangen und Analysieren von Urin nach einem der Ansprüche 1 bis 4,
- einen Behälter (167) zum Aufnehmen des urinierenden Tieres und zum Zurückhalten von gegenüber dem Urin inerten Elementen auf einem Sieb (168), das so konfiguriert ist, dass es die inerten Elemente zurückhält und den Urin durchlässt,
- eine geneigte Fläche unter dem Sieb, um den Urin in einem Sammelbereich zu sammeln, der zu einem Einlass des Behälters zum Auffangen und Analysieren von Urin führt.

6. Vorrichtung (160) nach Anspruch 5, wobei der Behälter (175) zumindest teilweise transparent ist und einen Tank (155) für entnommene Urinproben sowie einen Verschluss (181) für den Zugang zu diesem Tank umfasst.

7. Vorrichtung (160) nach einem der Ansprüche 5 oder 6, die zusätzlich eine Einrichtung (100, 110, 116, 119) zur Analyse des entnommenen Urins umfasst.

8. Vorrichtung (160) nach Anspruch 7, wobei die Analyseeinrichtung (100, 110, 116, 119) einen Lichtsensor (117) zum Erfassen der Farbe mindestens einer Kontrollprobe, die mindestens ein Reagenz enthält, und eine Einrichtung (118, 119) zum Ausgleichen von Beleuchtungsänderungen jeder Kontrollprobe umfasst.

9. Vorrichtung (160) nach Anspruch 8, wobei die Analyseeinrichtung (100, 110, 116, 119) eine Einrichtung (118, 119) zur Bewertung der Farbe des entnommenen Urins und/oder eine Einrichtung zur Bewertung der Klarheit des entnommenen Urins umfasst.

10. Verfahren zur Verwendung eines Behälters (175) nach einem der Ansprüche 1 bis 4 oder einer Vorrichtung (160) nach einem der Ansprüche 5 bis 9, das Folgendes umfasst:
- einen Schritt (131) zum Aufnehmen eines Bildes des Behälters,
- einen Schritt zum Identifizieren eines Streifens in dem aufgenommenen Bild,
- einen Schritt (134) zum Bestimmen der Farbe der auf dem Streifen befindlichen Punkte,
- einen Schritt (135 bis 137) zum Vergleichen mindestens einer Farbe der Punkte mit mindestens einem Farbgrenzwert, um eine Anomalie zu erkennen,
- einen Schritt zum Identifizieren mindestens eines Urinauffangbehälters im aufgenommenen Bild,
- einen Schritt zum Messen der Trübung und/oder Farbe des in mindestens einem Fach aufgefangenen Urins und
- einen Schritt zum Vergleichen der gemessenen Trübung und/oder Farbe mit mindestens einem Grenzwert, um eine Anomalie zu erkennen, und
- einen Schritt zum Anzeigen jeder erkannten Anomalie.

11. Verfahren nach Anspruch 10, das zusätzlich Folgendes umfasst:
- einen Schritt (130) zum Initialisieren von Grenzwerten für die Erkennung von Anomalien und
- einen Schritt zum Neubewerten mindestens eines Grenzwerts in Abhängigkeit von den für das betreffende Tier gemessenen Werten.

12. Verfahren nach einem der Ansprüche 10 oder 11, das zusätzlich Folgendes umfasst:
- einen ersten Schritt zur Bestimmung des Verhaltens des Tieres gegenüber dem Behälter, beispielsweise Uhrzeit, Dauer und Häufigkeit des Durchgangs,
- einen Schritt zur Initialisierung von Grenzwerten in Bezug auf das Verhalten des Tieres,
- einen zweiten Schritt zur Messung von Werten, die für das Verhalten des Tieres repräsentativ sind, und
- einen Schritt zum Vergleich der Messungen mit den Grenzwerten, um eine Anomalie zu erkennen.

13. Verfahren nach einem der Ansprüche 10 bis 12, das zusätzlich Folgendes umfasst:
- einen ersten Schritt zur Bestimmung des Gewichts des Tieres und/oder des Gewichts mindestens einer Urinausscheidung des Tieres,
- einen Schritt zur Initialisierung von Grenzwerten in Bezug auf das bestimmte Gewicht,
- einen zweiten Schritt zum Messen des Gewichts des Tieres und/oder des Gewichts mindestens einer Urinausscheidung des Tieres und
- einen Schritt zum Vergleichen der Messungen mit den Grenzwerten, um eine Anomalie zu erkennen.

14. Verfahren nach einem der Ansprüche 10 bis 13, das zusätzlich einen Schritt zum Erfassen der Farbe mindestens einer Kontrollprobe, die mindestens ein Reagenz enthält, und einen Schritt (133) zum Ausgleichen der Beleuchtungsunterschiede jeder Kontrollprobe umfasst.

## Claims

1. Unit (175) for capturing and analysing an animal's urine, which unit comprises:
- a first receptacle (188) for urine, and
- a second receptacle (191) for urine, equipped with a support (187) for a strip (195) bearing indicator pads containing reagents, said receptacles being separated by a partition (156) equipped with a passage (157), and at least one support (182) realising a tilting axis for switching the unit between:
- a first configuration wherein, when the passage is open, the urine flows by gravity towards the first receptacle, and
- a second configuration wherein, when the passage is open, the urine flows by gravity towards the second receptacle; and
- a valve (177) which closes or opens the passage between said receptacles;
said unit wherein:
- the first receptacle (188) comprises at least one lateral compartment (189, 190) and at least one partition (197, 198) for holding urine in a lateral compartment when the unit switches to the second configuration, and/or
- the device comprises a cover (185) that is transparent at least facing the support (187) for a strip (195), the cover being mobile relative to a base (184) comprising the receptacles (188, 191).

2. Unit (175) according to claim 1, wherein the valve (177) is a multiport valve which also closes or opens an inlet (176) for urine in the unit.

3. Unit (175) according to claim 2, wherein the valve (177) comprises a chamber (155) for holding the first stream of urine passing into the unit.

4. Unit (175) according to claim 3, wherein the chamber (155) for holding the first flow of urine is topped by a partition (154) limiting the flow of urine towards the second receptacle (191), in the second configuration of the unit.

5. Device (160) for collecting urine for a predefined animal, which device comprises:
- a unit (175) for capturing and analysing urine according to one of claims 1 to 4;
- a container (167) for receiving the urinating animal and for holding elements, inert with respect to the urine, on a screen (168) configured to hold the inert elements and let the urine pass;
- a sloping surface, under the screen, for collecting the urine in a convergence area leading to an inlet of the unit for capturing and analysing urine.

6. Device (160) according to claim 5, wherein the unit (175) is at least partially transparent and comprises a chamber for collected urine (155) and a cap (181) for access to this chamber.

7. Device (160) according to one of claims 5 or 6, which further comprises a means (100, 110, 116, 119) for analysing the urine collected.

8. Device (160) according to claim 7, wherein the analysis means (100, 110, 116, 119) comprises a light sensor (117) for capturing the colour of at least one indicator pad containing at least one reagent and a means (118, 119) for compensating for variations in lighting for each indicator pad.

9. Device (160) according to claim 8, wherein the analysis means (100, 110, 116, 119) comprises a means (118, 119) for assessing the colour of the urine collected and/or a means for assessing the clearness of the urine collected.

10. Method for using a unit (175) according to one of claims 1 to 4 or a device (160) according to one of claims 5 to 9, which method comprises:
- a step (131) of taking an image of the unit;
- a step of identifying a strip in the captured image;
- a step (134) of determining the colour of pads borne by the strip;
- a step (135 to 137) of comparing at least one pad colour with at least one colour limit value, for detecting an anomaly;
- a step of identifying at least one compartment holding the urine, in the captured image;
- a step de measuring the turbidity and/or colour of the urine held in at least one compartment; and
- a step of comparing the turbidity measured and/or the colour measured with at least one limit value for detecting an anomaly; and
- a step of displaying each anomaly detected.

11. Method according to claim 10, which further comprises:
- a step (130) of initialising limit values for detecting anomalies; and
- a step of reassessing at least one limit value on the basis of values measured for the animal considered.

12. Method according to one of claims 10 to 11, which further comprises:
- a first step of determining the behaviour of the animal with respect to the unit, for example the time, duration and frequency of passage;
- a step of initialising limit values in relation to the behaviour of the animal;
- a second step of measuring values representative of the behaviour of the animal; and
- a step of comparing measurements with the limit values, for detecting an anomaly.

13. Method according to one of claims 10 to 12, which further comprises:
- a first step of determining the weight of the animal and/or the weight of at least one urination of the animal;
- a step of initialising limit values in relation to the weight determined;
- a second step of measuring the weight of the animal and/or the weight of at least one urination of the animal; and
- a step of comparing measurements with the limit values, for detecting an anomaly.

14. Method according to one of claims 10 to 13, which further comprises a step of capturing the colour of at least one indicator pad containing at least one reagent and a step (133) of compensating for variations in lighting for each indicator pad.
